# EUROPEAN PATENT APPLICATION

(11) **EP 4 850 662 A1**
(43) Date of publication of application: **09.12.2026**
(21) Application number: 25748135.8
(22) Date of filing: 24.01.2025
(51) Int. Cl.: A61K 8/44, A61Q 19/00

(54) **AMINO ACID DERIVATIVE COMPOSITION, AND COSMETIC OBTAINED BY BLENDING SAME**

(30) Priority: 30.01.2024 JP 2024011714
(71) Applicant: Seiwa Kasei Company, Limited, Higashiosaka-shi, Osaka 579-8004 (JP)
(72) Inventor: YOSHIOKA Masato, Higashiosaka-shi, Osaka 579-8004 (JP); OYOBE Naoko, Higashiosaka-shi, Osaka 579-8004 (JP); IWAKI Nobuyasu, Higashiosaka-shi, Osaka 579-8004 (JP); HIROSE Shumpei, Higashiosaka-shi, Osaka 579-8004 (JP)
(74) Representative: Vossius & Partner Patentanwälte Rechtsanwälte mbB
(86) International application number: PCT/JP2025/002327
(87) International publication number: WO 2025/164553

(57) **Abstract**

The present invention provides: a composition characterized by containing an amino acid derivative represented by general formula (A), (B), or (C) or a salt thereof, wherein the content of component represented by formula (A) is 75-95 mass %, the content of component represented by formula (B) is 5-20 mass %, and the content of component represented by formula (C) is 0-1.5 mass %; and cosmetics comprising the composition and having excellent moisture retention and functionality while solving the problem of odors and degradation of the feel of use. 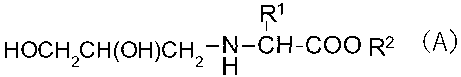 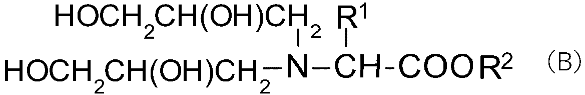 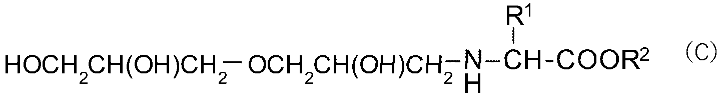 (In the formulas, R¹ represents a hydrogen atom or a side chain of an α-amino acid, and R² represents a hydrogen atom, alkali metal, ammonium, or organic ammonium.)

## Description

### [TECHNICAL FIELD]

The present invention relates to compositions obtained by combining amino acid derivatives or salts thereof in specific proportions, which are suitably used as raw materials for cosmetics, and to cosmetics obtained by blending these compositions.

### [BACKGROUND ART]

Amino acid derivatives are widely known as cosmetic ingredients with excellent functionality, and various studies have been conducted on them. For example, JP 5,625,914 B2 (Patent Document 1) discloses an amino acid derivative that can provide a cosmetic product that has good compatibility with skin and hair, high moisturizing properties, and a pleasant feel. However, when these amino acid derivatives are incorporated into cosmetics at high concentrations, odors derived from amino acids and a decrease in feel of use occur, so further improvements are required.

### [PRIOR ART DOCUMENTS]

### [PATENT DOCUMENT]

[Patent Document 1] JP 5,625,914 B2

### [SUMMARY OF INVENTION]

### [PROBLEM TO BE SOLVED BY THE INVENTION]

An object of the present invention is to solve problems such as odor and reduction of feel of use, and to provide amino acid derivative compositions and cosmetics containing them that have excellent moisturizing properties.

### [Means for solving the problem]

The inventors of this invention conducted extensive studies to solve the above problems and found that compositions containing amino acid derivatives represented by the following general formulas (A) to (C) or salts thereof in specific proportions exhibit superior moisturizing effects compared to compositions containing each of them individually. Furthermore, since cosmetics containing compositions with the above amino acid derivatives or salts thereof have excellent moisturizing effects, it is possible to reduce the use amount while maintaining equivalent moisturizing effects, thereby solving problems, such as odors derived from amino acids derivatives and a decrease in feel of use. This invention was completed based on these findings.

The first aspect of the present invention provides
a composition containing amino acid derivatives represented by the following general formulas (A), (B) and (C), or salts thereof, wherein component represented by the formula (A) is 75 to 95% by mass, component represented by the formula (B) is 5 to 20% by mass, and component represented by the formula (C) is 0 to 1.5% by mass.

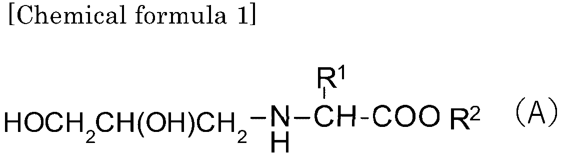

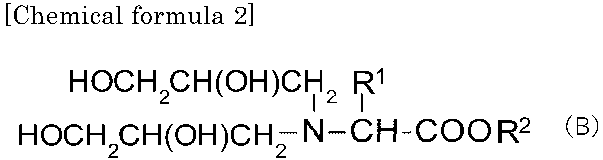

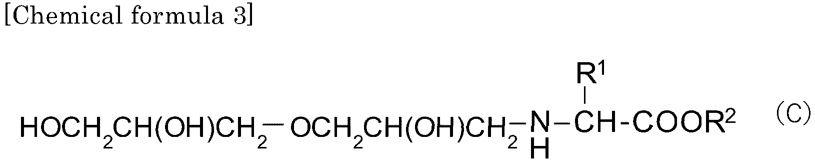

(In the formula (A), (B) and (C), R¹ represents a hydrogen atom or a side chain of an α-amino acid, and R² represents a hydrogen atom, alkali metal, ammonium, or organic ammonium. R¹ in formula (A), R¹ in formula (B), and R¹ in formula (C) are the same.)

The second aspect of the present invention is a preferred embodiment of the first aspect of the present invention, wherein the composition does not contain an amino acid derivative represented by the following general formula (D) and salts thereof, or if it does contain them, their content is 0.1% by mass or less. This composition is particularly preferred in having a high moisturizing effect.

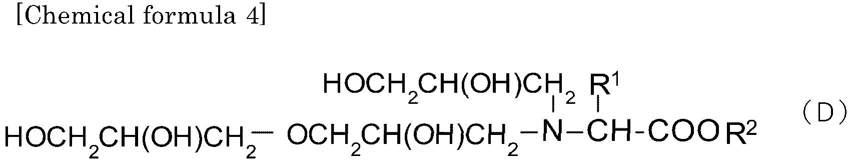

(In the formula, R¹ represents a hydrogen atom or a side chain of an α-amino acid, and R² represents a hydrogen atom, alkali metal, ammonium, or organic ammonium.)

The third aspect of the present invention is a preferred embodiment of the first or second aspect of the present invention, wherein the amino acid is arginine or glycine. Arginine or glycine are preferable because they provide an excellent feel of use when incorporated into cosmetics.

Compositions containing the amino acid derivatives or salts thereof of the present invention can be incorporated into cosmetics. The fourth aspect of the present invention is a cosmetic which incorporates the compositions of the first to third aspects of the present invention. The fourth aspect of the present invention is a cosmetic which exhibits a high moisturizing effect, reduces odor derived from amino acid derivatives, and provides an excellent feel.

### [EFFECT OF THE INVENTION]

Compositions according to the present invention, which contain amino acid derivatives represented by the general formula (A) to (C) or salts thereof in specific proportions exhibit superior moisturizing effects compared to compositions containing each individual component alone. Therefore, cosmetics containing these compositions can reduce the amount of each component used while maintaining the same moisturizing effect as those of conventional compositions containing amino acid derivatives or salts thereof, individually. This makes it possible to solve problems such as odor and reduction of feel of use when amino acid derivatives or salts thereof are incorporated into cosmetics.

### [EMBODIMENTS FOR CARRYING OUT THE INVENTION]

The following are embodiments for carrying out the present invention, but the scope of the present invention is not limited to the embodiments shown below.

Examples of amino acids used as raw materials (starting materials) for producing the amino acid derivatives represented by the general formulas (A) to (C) described above, or salts thereof, that constitute the composition of the present invention, include
acidic amino acids such as aspartic acid and glutamic acid, neutral amino acids such as isoleucine, leucine, valine, glycine, alanine, serine, threonine, phenylalanine, asparagine, glutamine, tyrosine, tryptophan, methionine, and cysteine, and
basic amino acids such as lysine, arginine, and histidine. Basic amino acids or neutral amino acids are preferred, and arginine or glycine are further preferred, as they are expected to exhibit an excellent feel of use when incorporated into cosmetics.

The amino acid derivatives represented by general formulas (A) to (C) or salts thereof that constitute the composition of the present invention can be obtained by reacting the amino acid or salt thereof with glycidol (i.e., 2,3-epoxy-1-propanol) or 3-halo-1,2-propanediol, followed by acetalization and acid treatment. Alternatively, they can be obtained by reacting the amino acid or salt thereof with 4-[(2,3-epoxypropoxy)methyl]-2,2-dimethyl-1,3-dioxolane or 4-halo-methyl-2,2-dimethyl-1,3-dioxolane, followed by acid treatment.

In the reaction with glycidol, 3-halo-1,2-propanediol, 4-[(2,3-epoxypropoxy)methyl]-2,2-dimethyl-1,3-dioxolane or 4-halo-methyl-2,2-dimethyl-1,3-dioxolane, the pH during the reaction is preferably 8 to 12, and more preferably 8 to 11. Examples of pH adjusting agents during the reaction include alkaline agents such as sodium hydroxide, potassium hydroxide, lithium hydroxide, sodium carbonate, and potassium carbonate.

The solvents used in the reaction include water, dimethylformamide, dimethyl sulfoxide, and N-methylpyrrolidone. The reaction temperature is preferably 10°C to 90°C, and more preferably 20°C to 80°C.

The compounds synthesized by the above method can be purified by means of column chromatography using silica gel or crystallization.

When reacting the amino acids or salts thereof with glycidol or 3-halo-1,2-propanediol, intermediates of the amino acid derivatives of the present invention, in which an acetal structure is introduced, can be obtained by reacting with various carbonyl compounds or dialkoxy compounds under acidic conditions.

Examples of carbonyl compounds used in the reaction include acetone, methyl ethyl ketone, diethyl ketone, cyclopentanone, cyclohexanone, formaldehyde, acetaldehyde, and benzaldehyde.

Examples of dialkoxy compounds used in the reaction include 2,2-dimethoxypropane, 2-methoxy-2-ethoxypropane, 2,2-diethoxypropane, and α,α-dimethoxytoluene.

The pH during the reaction is preferably 1 to 5, more preferably 2 to 4. Examples of pH adjusting agents include acids such as p-toluenesulfonic acid, methanesulfonic acid, hydrochloric acid, and sulfuric acid.

Examples of solvents used in the reaction include alcohols, acetone, and tetrahydrofuran. The reaction temperature is preferably between 0°C and 40°C, and more preferably between 10°C and 30°C.

The intermediate of the amino acid derivative contained in the present invention, synthesized by the above method, into which the acetal structure has been introduced, can be purified by means of column chromatography using silica gel or crystallization.

The intermediate of the amino acid derivative contained in the present invention, synthesized by the above method, into which the acetal structure has been introduced, and the reaction product of an amino acid or a salt thereof with 4-[(2,3-epoxypropoxy)methyl]-2,2-dimethyl-1,3-dioxolane or 4-halo-methyl-2,2-dimethyl-1,3-dioxolane, can be subjected to acid treatment under acidic conditions in the presence of water to obtain amino acid derivatives represented by general formulas (A) to (C) or salts thereof that constitute the composition of the present invention.

The pH during the reaction is preferably 1 to 5, and more preferably 1 to 3. Examples of pH adjusting agents during the reaction include acids such as p-toluenesulfonic acid, methanesulfonic acid, hydrochloric acid, and sulfuric acid.

Examples of solvents used in the reaction include water, alcohol, and tetrahydrofuran. The reaction temperature is preferably between 0°C and 40°C, and more preferably between 10°C and 30°C.

The amino acid derivatives represented by the general formulas (A) to (C) described above or salts thereof, which constitute the composition of the present invention synthesized by the above method, can be purified by means of column chromatography using silica gel or crystallization.

Furthermore, the amino acid derivative may also be in salt form, and examples of the salt form include sodium salt, potassium salt, calcium salt, alkylamine salt, hydrochloride salt, sulfate salt, phosphate salt, and acetate salt.

The proportions of the composition of the present invention are 75 to 95% by mass of component (A), 5 to 20% by mass of component (B), and 0 to 1.5% by mass of component (C). If the proportions deviate from the above, the moisturizing effect will decrease, which is undesirable. A more preferable proportion is 80 to 90% by mass of component (A), 8 to 18% by mass of component (B), and 0.5 to 1.5% by mass of component (C).

The compositions containing components represented by general formulas (A) to (C) of the present invention preferably do not contain the amino acid derivative represented by general formula (D) or salts thereof, or if they do, contain 0.1% by mass or less of (D). Within this range, it is preferable because it exhibits a particularly high moisturizing effect when incorporated into cosmetics.

The mixtures (compositions) of the amino acid derivatives or salts thereof of the present invention can be applied to cosmetics, and, as long as stability and other properties are not impaired, the cosmetics may contain any ingredients commonly used in cosmetics, depending on their intended use.

Ingredients commonly used in cosmetics may be appropriately blended, and examples of the ingredients include oily components, surfactants, emulsifiers, polymer compounds such as thickeners, whitening agents, texture enhancers, pharmaceuticals, UV absorbers, proteins, protein hydrolysates or their derivatives, amino acids or amino acid derivatives other than components (A) to (C), antioxidants, metal ion chelating agents, pH adjusters, preservatives, humectants, pigments, colorants, and fragrances.

Examples of oily components, surfactants, emulsifiers, polymer compounds such as thickeners, whitening agents, texture enhancers, pharmaceuticals, UV absorbers, proteins, protein hydrolysates or their derivatives, amino acids or amino acid derivatives other than components (A) to (C), antioxidants, metal ion chelating agents, pH adjusters, preservatives, humectants, pigments, colorants, fragrances, etc., are similar to those described in WO 2022/080287 A1.

The above-mentioned ingredients commonly used in the cosmetics can be used individually or in combination of two or more.

### [EXAMPLES]

Next, the present invention will be specifically described with reference to synthesis examples and embodiments. However, the present invention is not limited to these synthesis examples and embodiments.

### Synthesis Example 1: Synthesis of N-glycerylarginine

Into a round-bottom flask, arginine (10.45 g), deionized water (75 ml), and sodium hydroxide (0.60 g) were added, and the temperature was raised to 40°C. To the mixture, glycidol (1.11 g) was added over 1 hour, followed by stirring for 3 hours. After neutralization with 17% hydrochloric acid, the mixture was concentrated under reduced pressure. To the resulting residue (12.31 g), acetone (75 ml), p-toluenesulfonate sodium monohydrate (3.00 g), and 2,2-dimethoxypropane (9.00 g) were added, and the mixture was stirred overnight at room temperature. Then, after adding 25% sodium hydroxide aqueous solution, the mixture was concentrated under reduced pressure. The resulting residue (20.54 g) was subjected to silica gel column chromatography, eluted with a chloroform/methanol/deionized water = 10:10:3 solution, and concentrated under reduced pressure to obtain N-4-methyl-2,2-dimethyl-1,3-dioxolanylarginine.

Further, to the N-4-methyl-2,2-dimethyl-1,3-dioxolanylarginine (0.75 g), 3.83 g of 5% hydrochloric acid was added, and the mixture was stirred overnight at room temperature. Then, after neutralization with 25% aqueous sodium hydroxide solution, the mixture was concentrated under reduced pressure. Subsequently, the resulting residue (0.56 g) was subjected to silica gel column chromatography, eluted with a chloroform/methanol/deionized water = 10:10:3 solution, and concentrated under reduced pressure to obtain N-glycerylarginine (yield 22.8%), which has the structure shown below.

The product thus obtained was subjected to mass spectrometry, ¹H-NMR, and ¹³C-NMR measurements, and was confirmed to be N-glycerylarginine represented by the structural formula shown below. In the synthesis examples shown below, the obtained products were measured similarly, and confirmed to be amino acid derivatives represented by the structural formulas and compound names shown in each synthesis example. The mass spectrometry, ¹H-NMR, and ¹³C-NMR measurement results of the products obtained in the following synthesis examples are shown in Tables 1 to 3.

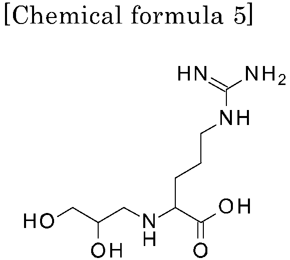

### Synthesis Example 2: Synthesis of N,N-Diglycerylarginine

Into a round-bottom flask, arginine (2.09 g), deionized water (15 ml), and sodium hydroxide (0.96 g) were added, and the temperature was raised to 40°C. To the mixture, glycidol (1.78 g) was added over 1 hour, followed by stirring for 3 hours. After that, 17% hydrochloric acid was added to neutralize the mixture, and then the solution was concentrated under reduced pressure. To the resulting residue (5.11 g), acetone (40 ml), p-toluenesulfonate sodium monohydrate (1.00 g), and 2,2-dimethoxypropane (5.50 g) were added, and the mixture was stirred overnight at room temperature. Then, after adding 25% sodium hydroxide aqueous solution, the mixture was concentrated under reduced pressure. The resulting residue (11.38 g) was subjected to silica gel column chromatography, eluted with a chloroform/methanol/deionized water = 7:4:0.5 solution, and concentrated under reduced pressure to obtain N,N-di-(4-methyl-2,2-dimethyl-1,3-dioxolanyl)arginine.

Further, to the N,N-di-(4-methyl-2,2-dimethyl-1,3-dioxolanyl) arginine (0.20 g), 5% hydrochloric acid (2.00 g) was added, and the mixture was stirred overnight at room temperature. Then, after neutralization with 25% aqueous sodium hydroxide solution, the mixture was concentrated under reduced pressure. Subsequently, the resulting residue (0.39 g) was subjected to silica gel column chromatography, eluted with a chloroform/methanol/ion-exchanged water = 10:10:3 solution, and concentrated under reduced pressure to obtain N,N-diglycerylarginine (yield 11.0%), which has the structure shown below.

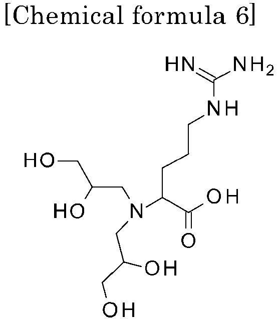

### Synthesis Example 3: Synthesis of N-(O-glyceryl) glycerylarginine

Into a round-bottom flask, arginine (0.66 g) and deionized water (2.2 ml) were added and the temperature was raised to 50°C. Then, 4-[(2,3-epoxypropoxy)methyl]-2,2-dimethyl-1,3-dioxolane (0.72 g) was added over 15 minutes, followed by stirring for 1.5 hours and concentration under reduced pressure. The resulting residue (1.48 g) was subjected to silica gel column chromatography, eluted with methanol, and concentrated under reduced pressure to obtain N-(O-4-methyl-2,2-dimethyl-1,3-dioxolanyl) glycerylarginine.

Further, to the N-(O-4-methyl-2,2-dimethyl-1,3-dioxolanyl) glycerylarginine (0.36 g), 1.00 g of 5% hydrochloric acid was added, and the mixture was stirred overnight at room temperature. Then, after neutralization with 25% aqueous sodium hydroxide solution, the mixture was concentrated under reduced pressure. The resulting residue (0.31 g) was subjected to silica gel column chromatography, eluted with methanol, and concentrated under reduced pressure to obtain N-(O-glyceryl) glycerylarginine (yield 90.0%), which has the structure shown below.

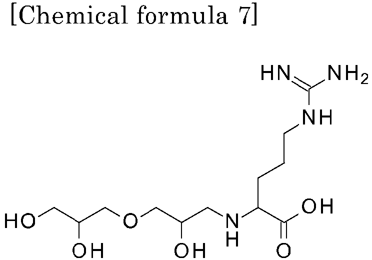

### Synthesis Example 4: Synthesis of N-glycerylglycine

Into a round-bottom flask, glycine (10.0 g), deionized water (30 ml), N,N-dimethylformamide (10 ml), sodium hydroxide (2.40 g), and 4-chloromethyl -2,2- dimethyl -1,3- dioxolane (9.79 g) were added, and the mixture was stirred overnight at 80°C. Then, after neutralization with 17% hydrochloric acid, the mixture was concentrated under reduced pressure. The resulting residue (23.01 g) was subjected to silica gel column chromatography, eluted with a chloroform/methanol/deionized water = 6:4:1 solution, and concentrated under reduced pressure to obtain N-4-methyl-2,2-dimethyl-1,3-dioxolanylglycine.

Further, to the N-4-methyl-2,2-dimethyl-1,3-dioxolanylglycine (0.54 g), 5% hydrochloric acid (2.80 g) was added, and the mixture was stirred overnight at room temperature. Then, after neutralization with 1% aqueous sodium hydroxide solution, the mixture was concentrated under reduced pressure. Subsequently, the resulting residue (0.79 g) was subjected to silica gel column chromatography, eluted with a chloroform/methanol /deionized water = 6:4:1 solution, and concentrated under reduced pressure to obtain N-glycerylglycine (yield 21.2%), which has the structure shown below.

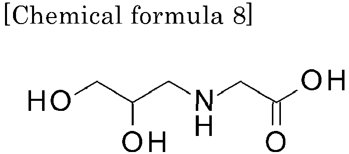

### Synthesis Example 5: Synthesis of N,N-Diglycerylglycine

Into a round-bottom flask, glycine (2.50 g), deionized water (10 ml), and sodium hydroxide (4.00 g) were added and the temperature was raised to 40°C. Then, glycidol (7.41 g) was added over 1 hour, followed by stirring for 3 hours. After neutralization with 17% hydrochloric acid, the mixture was concentrated under reduced pressure.
To the resulting residue (18.28 g), acetone (200 ml), p-toluenesulfonate sodium monohydrate (4.75 g), and 2,2-dimethoxypropane (50.00 g) were added and stirred overnight at room temperature. Then, after adding 1% sodium hydroxide aqueous solution, the mixture was concentrated under reduced pressure. The resulting residue (23.97 g) was subjected to silica gel column chromatography, eluted with a chloroform/methanol/deionized water = 10:3:0.3 solution, and concentrated under reduced pressure to obtain N,N-di-(4-methyl-2,2-dimethyl-1,3-dioxolanyl)glycine.

Further, to the N,N-di-(4-methyl-2,2-dimethyl-1,3-dioxolanyl)glycine (0.18 g), 5% hydrochloric acid (0.60 g) was added, and the mixture was stirred overnight at room temperature. Then, after neutralization with 1% aqueous sodium hydroxide solution, the mixture was concentrated under reduced pressure. The resulting residue (0.36 g) was subjected to silica gel column chromatography, eluted with a chloroform/methanol/deionized water = 6:4:1 solution, and concentrated under reduced pressure to obtain N,N-diglycerylglycine (yield 30.2%), which has the structure shown below.

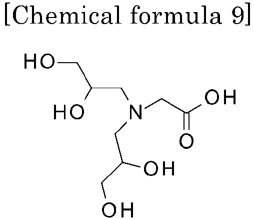

### Synthesis Example 6: Synthesis of N-(O-glyceryl)glycerylglycine

Into a round-bottom flask, glycine (0.38 g), 25% sodium hydroxide aqueous solution (0.16 g), and deionized water (1.5 ml) were added and the temperature was raised to 50°C. Then, 4-[(2,3-epoxypropoxy) methyl]-2,2-dimethyl-1,3-dioxolane (0.94 g) was added over 15 minutes, followed by stirring for 3 hours and concentration under reduced pressure. The resulting residue (1.17 g) was subjected to silica gel column chromatography, eluted with a chloroform/methanol/deionized water = 5:4:0.4 solution, and then concentrated under reduced pressure to obtain N-(O-4-methyl-2,2-dimethyl-1,3-dioxolanil)glycerylglycine.

Further, to the N-(O-4-methyl-2,2-dimethyl-1,3-dioxolanyl) glycerylglycine (0.53 g), 2.00 g of 5% hydrochloric acid was added, and the mixture was stirred overnight at room temperature. Then, after neutralization with 1% aqueous sodium hydroxide solution, the mixture was concentrated under reduced pressure. The resulting residue (0.42 g) was subjected to silica gel column chromatography, eluted with a chloroform/methanol/deionized water = 1:1:0.1 solution, and concentrated under reduced pressure to obtain N-(O-glyceryl)glycerylglycine (yield 91.0%), which has the structure shown below.

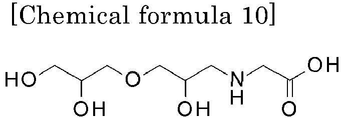

Mass spectrometry was performed on the products obtained in Synthesis Examples 1 to 6 using LCMS-2020 (Manufactured by Shimadzu Corporation). The measurement results are shown in Table 1.

**[Table 1]**

| Synthesis example number | Ionization | Measured values |
|---|---|---|
| 1 | [M+H] ⁺ | 249.3 |
| 2 | [M+H] ⁺ | 323.4 |
| 3 | [M+H] ⁺ | 323.4 |
| 4 | [M+H] ⁺ | 150.2 |
| 5 | [M+H] ⁺ | 224.2 |
| 6 | [M+H] ⁺ | 224.2 |

| | | |
|---|---|---|
| * Ionization was performed using electrospray ionization (ESI). | | |

¹H-NMR measurements of the products obtained in Synthesis Examples 1 to 6 were performed using a JNM-ECS400 (manufactured by JEOL Ltd.). The measurement results are shown in Table 2.

**[Table 2]**

| Synthesis example number | Measurement conditions | Chemical shift δ (ppm) |
|---|---|---|
| 1 | 400MHz, D₂O | 1.53-1.74(2H, m), 1.82-2.00(2H, m), 3.18(1H, t), 3.20(2H, t), 3.50-3.72(4H, m), 4.00(1H, m) |
| 2 | 400MHz, D₂O | 1.75(2H, m), 1.90(2H, m), 3.25(1H, t), 3.27(2H, t), 3.51-3.67(8H, m), 3.90(1H, br ), 4.11(1H, br ) |
| 3 | 400MHz, D₂O | 1.59-1.80(4H, br ), 3.19(1H, t), 3.20(2H, t), 3.48-3.64(8H, m), 3.88(2H, m) |
| 4 | 400MHz, CD₃OD | 3.05(1H, dd), 3.21(1H, dd), 3.53-3.62(4H, m), 3.94(1H, m) |
| 5 | 400MHz, CD₃OD | 3.26-3.33(4H, m), 3.39-3.62(8H, m), 3.76(1H, dd), 3.87(1H, m), 3.90(1H, dd), 4.05(1H, br ) |
| 6 | 400MHz, CD₃OD | 3.08(1H, dd), 3.18(1H, dd), 3.46-3.61(8H, m), 3.79(1H, m), 4.05(1H, m) |

¹³C-NMR measurements of the products obtained in Synthesis Examples 1 to 6 were performed using JNM-ECS400 (manufactured by JEOL Ltd.). The measurement results are shown in Table 3.

**[Table 3]**

| Synthesis example number | Measurement conditions | Chemical shift δ (ppm) |
|---|---|---|
| 1 | 100 MHz, D₂O | 23.9, 26.5, 40.5, 48.9, 49.0, 63.3, 67.1, 156.7, 173.2 |
| 2 | 100 MHz, D₂O | 25.2, 25.8, 40.8, 49.0, 55.5, 62.5, 63.2, 63.3, 70.4, 70.4, 156.8, 172.1 |
| 3 | 100 MHz, D₂O | 25.7, 26.3, 40.7, 48.9, 55.4, 63.3, 67.8, 156.7, 171.9 |
| 4 | 100 MHz, CD₃OD | 50.7, 51.5, 65.2, 68.6, 171.3 |
| 5 | 100 MHz, CD₃OD | 58.6, 59.6, 65.1, 68.1, 170.7 |
| 6 | 100 MHz, CD₃OD | 51.1, 51.9, 64.2, 67.2, 72.0, 74.0, 74.8, 171.9 |

### Test Example 1 [Moisture Test]

Each sample, prepared in the proportions shown in Examples 1-12 and Comparative Examples 1-6 in Tables 4 and 5 below, was vacuum-dried, and approximately 40 mg (this weight is denoted as W₀) of each sample was placed in a weighing bottle (3.6 cm in diameter, 1.8 cm in height excluding the lid). Each sample in the weighing bottle was left to stand in a constant temperature and humidity chamber (LTI-1200, manufactured by Tokyo Rikakikai Co., Ltd.) under an environment at a temperature of 25°C and a humidity of 75%. The weight was measured periodically, and the samples were left to stand until sufficient moisture had been absorbed and the weight no longer changed. After that, the samples were transferred to an environment at a temperature of 25°C and a humidity of 20% (in a sealed container filled with saturated potassium acetate aqueous solution at the bottom), and the amount of moisture retained per gram of dry sample Δ (g/g) was calculated from the weight after 24 hours (this weight is denoted as W₁) using the following formula. $\left({W}_{1}-{W}_{0}\right) / {W}_{0}$

**[Table 4]**

| Example Number | Synthesis Example 1 (%) | Synthesis Example 2 (%) | Synthesis Example 3 (%) | Moisture retention amount Δ (g/g) |
|---|---|---|---|---|
| Comparison 1 | 100 | 0 | 0 | 0.060 |
| Comparison 2 | 0 | 100 | 0 | 0.045 |
| Comparison 3 | 0 | 0 | 100 | 0.028 |
| 1 | 90 | 10 | 0 | 0.080 |
| 2 | 80 | 20 | 0 | 0.091 |
| 3 | 90 | 9.5 | 0.5 | 0.081 |
| 4 | 80 | 19.5 | 0.5 | 0.088 |
| 5 | 90 | 9.0 | 1.0 | 0.072 |
| 6 | 80 | 19.0 | 1.0 | 0.074 |

**[Table 5]**

| Example Number | Synthesis Example 4 (%) | Synthesis Example 5 (%) | Synthesis Example 6 (%) | Moisture retention amount Δ (g/g) |
|---|---|---|---|---|
| Comparison 4 | 100 | 0 | 0 | 0.040 |
| Comparison 5 | 0 | 100 | 0 | 0.033 |
| Comparison 6 | 0 | 0 | 100 | 0.019 |
| 7 | 90 | 10 | 0 | 0.068 |
| 8 | 80 | 20 | 0 | 0.073 |
| 9 | 90 | 9.5 | 0.5 | 0.066 |
| 10 | 80 | 19.5 | 0.5 | 0.071 |
| 11 | 90 | 9.0 | 1.0 | 0.062 |
| 12 | 80 | 19.0 | 1.0 | 0.068 |

As shown in Tables 4 and 5, compositions of the present invention containing the amino acid derivatives of the general formulas (A) to (C) or salts thereof, which are mixed in specific proportions, have been shown to exhibit superior moisturizing effects (water retention) compared to the amino acid derivatives of the general formulas (A) to (C) or salts thereof alone. In other words, compositions of the present invention containing the amino acid derivatives or salts thereof make it possible to reduce the amount of amino acid derivatives or salts thereof used in cosmetics while maintaining their moisturizing ability, thereby suppressing the odor derived from the amino acid derivatives and deterioration of the feel of use of the product.

### Example 13: Lotion

A lotion is prepared by mixing the raw materials (1) to (6) shown in Table 6 while stirring well. In Table 6 and subsequent Tables, the amounts are expressed in parts by mass.

**[Table 6]**

| No | Ingredient name | Blend amount |
|---|---|---|
| 1 | Amino acid derivative composition of Example 2 | 7.0 |
| 2 | ethanol | 8.0 |
| 3 | citric acid | 0.01 |
| 4 | Sodium citrate | 0.015 |
| 5 | Potassium glycyrrhizinate | 0.03 |
| 6 | purified water | remainder *1 |

| | | |
|---|---|---|
| *1: This refers to the amount required to make a total amount of 100 parts by mass. The same applies to the following tables. | | |

### Example 14 Emulsion

The raw materials for the oil phase (1) to (9) and the raw materials for the aqueous phase (10) to (13), shown in Table 7, are heated to 70°C and dissolved to prepare the oil phase and aqueous phase, respectively. Then, the oil phase is added to the aqueous phase and pre-emulsified. After uniform emulsification with a homo-mixer, the mixture is cooled to room temperature while stirring well to prepare an emulsion.

**[Table 7]**

| No | Ingredient name | Blend amount |
|---|---|---|
| 1 | Isostearyl palmitate | 5.0 |
| 2 | Jojoba oil | 2.0 |
| 3 | Dimethylpolysiloxane | 2.0 |
| 4 | Cetanol | 1.0 |
| 5 | Stearic acid | 1.5 |
| 6 | Beeswax | 2.5 |
| 7 | Paraffin wax | 2.5 |
| 8 | Polyoxyethylene (20) sorbitan monostearate | 1.2 |
| 9 | Polyoxyethylene (40) sorbitol tetraoleate | 1.5 |
| 10 | Propylene glycol | 10.0 |
| 11 | Composition of Example 6 | 3.0 |
| 12 | Preservatives | appropriate amount |
| 13 | purified water | remainder |

### Example 15 Cream

The raw materials for the oil phase (1) to (5) and the raw materials for the aqueous phase (6) to (10), shown in Table 8, are heated to 70°C and dissolved to prepare the oil phase and aqueous phase, respectively. Then, the oil phase is added to the aqueous phase and pre-emulsified. After uniform emulsification with a homo-mixer, the mixture is cooled to room temperature while stirring well to prepare a cream.

**[Table 8]**

| No | Ingredient name | Blend amount |
|---|---|---|
| 1 | Squalane | 9.0 |
| 2 | Vaseline | 6.0 |
| 3 | Stearyl alcohol | 5.0 |
| 4 | Polyoxyethylene (25) cetyl ether | 2.5 |
| 5 | Glyceryl monostearate | 1.5 |
| 6 | Composition of Example 8 | 2.0 |
| 7 | Glycerin | 6.0 |
| 8 | Preservatives | appropriate amount |
| 9 | pH adjuster | appropriate amount |
| 10 | purified water | remainder |

## Claims

1. A composition containing amino acid derivatives represented by the following general formulas (A), (B) and (C), or salts thereof, wherein component represented by the formula (A) is 75 to 95% by mass, component represented by the formula (B) is 5 to 20% by mass, and component represented by the formula (C) is 0 to 1.5% by mass. 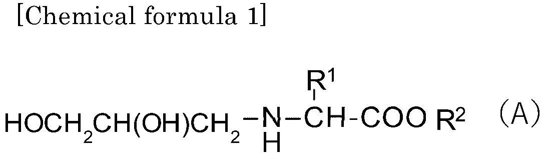 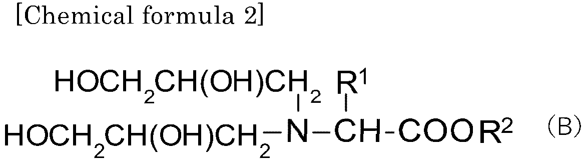 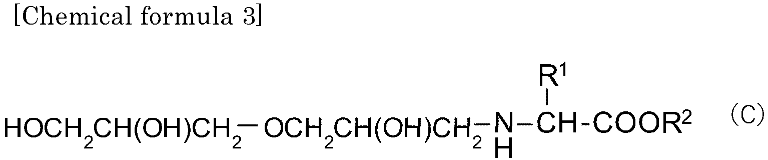 (In the formulas (A), (B) and (C), R¹ represents a hydrogen atom or a side chain of an α-amino acid, and R² represents a hydrogen atom, alkali metal, ammonium, or organic ammonium. R¹ in formula (A), R¹ in formula (B), and R¹ in formula (C) are the same.)

2. The composition according to claim 1, **characterized in that** it does not contain amino acid derivatives represented by the following general formula (D) and salts thereof, or if it does contain them, their content is 0.1% by mass or less. 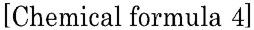 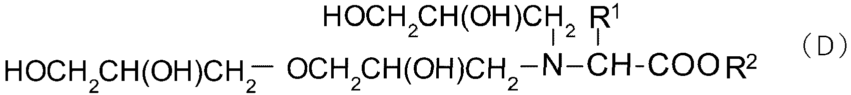 (In the formula, R¹ represents a hydrogen atom or a side chain of an α-amino acid, and R² represents a hydrogen atom, alkali metal, ammonium, or organic ammonium.)

3. The composition according to claim 1 or 2, wherein the amino acid is arginine or glycine.

4. A cosmetic composition which contains the composition according to any one of claims 1 to 3.
